(19) Europäisches Patentamt
European Patent Office
Office européen des brevets

(11) **EP 1 813 188 B1**

(12) **EUROPÄISCHE PATENTSCHRIFT**

(45) Veröffentlichungstag und Bekanntmachung des Hinweises auf die Patenterteilung:
**30.03.2011 Patentblatt 2011/13**

(51) Int Cl.:
***A61B 5/0275*** *(2006.01)*   ***A61M 1/36*** *(2006.01)*

(21) Anmeldenummer: **06101019.5**

(22) Anmeldetag: **30.01.2006**

(54) **System zum Einrichten einer Dilutionsmessstelle**

System for providing a dilution measuring point

Système pour fournir un point de mesure de la dilution

(84) Benannte Vertragsstaaten:
**DE ES FR GB IT**

(43) Veröffentlichungstag der Anmeldung:
**01.08.2007 Patentblatt 2007/31**

(73) Patentinhaber: **Pulsion Medical Systems AG**
**81829 München (DE)**

(72) Erfinder:
• **Goedje, Oliver, Dr.**
**82031 Grünwald (DE)**
• **Thalmeier, Thomas**
**84405 Dorfen (DE)**

• **Bohn, Matthias**
**81477 München (DE)**

(74) Vertreter: **Ettmayr, Andreas et al**
**Kehl & Ettmayr**
**Patentanwälte**
**Friedrich-Herschel-Straße 9**
**81679 München (DE)**

(56) Entgegenhaltungen:
**EP-A- 0 693 297    WO-A-87/04079**
**WO-A-2005/049113    DE-A1- 10 143 995**
**DE-C1- 19 528 907    US-A- 5 595 182**

**Beschreibung**

Die vorliegende Erfindung betrifft eine Vorrichtung zum Einrichten einer Dilutionsmeßstelle an einem Hämodialysator.

Allein in Deutschland müssen sich aufgrund von Nierenerkrankungen etwa 50000 Patienten regelmäßig, d.h. mehrmals wöchentlich, einer Hämodialyse (Blutwäsche) unterziehen. Dabei werden, wie hinlänglich bekannt ist, extrakorporale Hämodialysatoren (sogenannte "künstliche Nieren") direkt an den Blutkreislauf des betroffenen Patienten angeschlossen. Der Anschluß erfolgt meist über Fistula-Nadeln an einer arteriovenösen Fistel, d.h. einer chirurgisch geschaffenen Gefäßverbindung zwischen einer Arterie und einer Vene, oder aber über einen sogenannten Shunt, d.h. eine extrakorporale künstliche Verbindungsleitung, welche mit einer Arterie und einer Vene verbunden wird. Dabei wird Blut von der arteriellen Seite dem Hämodialysator zugeführt und auf der venösen Seite in den Körper rückgeführt. Hämodialysatoren weisen neben Pumpen, Wärmetauschern und einer Luftfalle, welche das venenseitige Eintreten von Luftbläschen in den Körperkreislauf verhindert, als Herzstück den eigentlichen Dialysator auf. Dieser besitzt große, beispielsweise als Kapillar- oder Foliensystem ausgeführte Membranflächen, welche auf einer Membranseite von Patientenblut und auf der anderen Membranseite von einer Dialysierflüssigkeit überströmt werden. Auf Grundlage diffusiven Austauschs durch die Membranflächen werden zu hoch konzentrierte Elektrolyte und harnpflichtige Substanzen dem Blut entzogen. Ferner findet ein Wasseraustausch statt, und es können gegebenenfalls Glucose und unterkonzentrierte Elektrolyte zugeführt werden.

Hämodialysatoren sind mit komplizierten Bilanzierungssystemen ausgestattet, welche unter anderem vermeiden sollen, daß über Wassergehaltsänderungen des dialysierten Bluts dem Körper des angeschlossenen Patienten zuviel Flüssigkeit entzogen oder zugeführt wird. Für Wohlbefinden und Gesundheit des Patienten ist es entscheidend, daß dessen Kreislauffüllungszustand, d.h. das gesamte zirkulierene Blutvolumen seines Körpers, einen Sollbereich nicht über und vor allem nicht unterschreitet. Hierfür werden, neben dem Einsatz des besagten Bilanzierungssystems, die Patienten jeweils vor und nach der Dialysebehandlung genau gewogen. Dennoch ist eine exakte Bilanzierung schwierig, da Dialysepatienten während der sich über mehrere Stunden hinziehenden Dialysebehandlungen häufig feste und oder flüssige Nahrung zu sich nehmen sowie Ausscheidungen in Form von Schweiß, Urin und Kot auftreten können.

Diesbezüglich eine Verbesserung zu schaffen, d.h. eine den Dialysepatienten wie auch das betroffene medizinische Personal wenig beanspruchende, gut zu handhabende, funktionssichere und komfortable Beurteilung des Kreislauffüllzustands von Dialysepatienten zu ermöglichen, ist Aufgabe der vorliegenden Erfindung.

Gelöst wird diese Aufgabe gemäß einem Aspekt der vorliegenden Erfindung durch eine Vorrichtung zur Einrichtung einer Dilutionsmeßstelle an einem Hämodialysator. Mit einer solchen Meßstelle ist die Möglichkeit gegeben, mittels Dilutionsmessungen den Kreislauffüllzustand eines Dialysepatienten auch zu beliebigen Zeitpunkten während der Dialysebehandlung, insbesondere jedoch unmittelbar nach deren Beginn und vor deren Ende zu bestimmen. Das fehleranfällige Überprüfen der Flüssigkeitsbüanz mittels Wiegen des Dialysepatienten kann entfallen. Die Einrichtung einer Dilutionsmeßstelle mittels einer erfindungsgemäßen Vorrichtung ermöglicht nicht nur die Ermittlung des Füllzustands sondern auch die Bestimmung anderer hämodynamischer Parameter mittels bekannter Thermo- oder Indikator-Dilutionstechniken. Insbesondere für die Überwachung von auf die Hämodialyse angewiesenen Intensivpatienten ergeben sich so Vorteile einer einfachen, sicheren, und komfortablen Ermittlung relevanter Daten.

Ein Verfahren zum Bestimmen des Kreislauffüllungszustandes eines Patienten mittels Thermodilutionsmessungen, für dessen Ausführung eine erfindungsgemäß eingerichtete Meßstelle auf besonders vorteilhafte Weise nutzbar ist, ist in der deutschen Offenlegungsschrift DE 42 14 402 A1 beschrieben. Ein Verfahren zum Bestimmen des zirkulierenden Blutvolumens eines Patienten mittels Indikatordilutionsmessungen, für dessen Ausführung ebenfalls eine erfindungsgemäß eingerichtete Meßstelle nutzbar ist, ist aus der deutschen Offenlegungsschrift DE 41 30 93 A1 bekannt. Auch für ein Indikatordilutionsmeßverfahren entsprechend oder ähnlich der US-Patentschrift 6,757,554 B2 oder für ein kombiniertes Indikator- und Thermodilutions-Meßverfahren (Doppelindikatordilutionstechnik), wie in der deutschen Offenlegungsschrift DE 101 43 995 A1 beschrieben, ist eine erfindungsgemäß eingerichtete Meßstelle nutzbar.

Aus der US Patentschrift 5,595,182 ist weiterhin ein System zur Vornahme von Dilutionsmessungen am Herzkreislaufsystem eines Dialysepatienten bekannt welches folgende Merkmale aufweist: ein venöses Anschlußstück mit einem Hamodialysatorausgangsanschluß und einem Injektionskanal; ein arterielles Anschlußstück mit einem Hamodialysatoreingangsanschluß und einen Sensor zur Messung der Temperatur zu dialysierenden Bluts; sowie eine Auswerteeinheit, welche einen Eingangskanal zum Einlesen eines Meßsignals des Sensors zur Messung der Temperatur zu dialysierenden Bluts sowie einen weiteren Eingangskanal zum Einlesen einer gemessenen Schallgeschwindigkeit des Blutes aufweist, wobei die Auswerteeinheit programmtechnisch dazu ausgelegt ist, eine Dilutionsmessung anhand der Schallgeschwindigkeit durchzuführen, die mittels der Temperatur korrigiert wird.

Insbesondere wird die der vorliegenden Erfindung zugrundeliegende Aufgabe durch die Systeme gemäß den Ansprüchen 1, 7 gelöst. Besonders vorteilhafte Ausführungsformen können gemäß einem der Ansprüche 2-6, 8-18 gestaltet sein.

Wie oben erläutert, eignet sich die erfindungsgemäße Vorrichtung sowohl für den vorteilhaften Einsatz der Thermodilutionstechnik als auch der Indikatordilutionstechnik oder einer Kombination hieraus.

Arterielles und venöses Anschlußstück können vorteilhafterweise mechanisch verbunden und somit in eine gemeinsame Funktionseinheit integriert sein, jedoch auch eine separate Ausführung der Anschlußstücke ist vorteilhaft umsetzbar. Dabei ist die Integration von arteriellem und venösem Anschlußstück in einen Shunt ebenso möglich, wie die Verbindung von arteriellem und venösem Anschlußstück mit Fistula-Nadeln. Diese Verbindung kann vorteilhafterweise sowohl fest, als auch lösbar, beispielsweise mittels Luer-Lock-Verbindungen, ausgeführt sein. Auch für die, in der Regel lösbare, Verbindung zum Hämodialysator an Hämodialysatoreingangsanschluß und Hämodialysatorausgangsanschluß können vorteilhafterweise Luer-Lock-Verbindungen aber auch anderweitig ausgeführte Schraub-, Bajonett-, Einrast-, Klemmverbindungen etc. vorgesehen werden.

Insbesondere der arterielle Gefäßanschluß (grundsätzlich aber auch der venöse Gefäßanschluß) kann ebenso mittels eines Katheters realisiert werden. Dabei besteht die Möglichkeit, den Sensor zur Messung der Temperatur des zu dialysierenden Bluts und/oder den Sensor zur Messung der Indikatorkonzentration im zu-dialysierendem Blut vorteilhafterweise in den Katheter zu integrieren oder mittels einer Sonde durch den Katheter-einzuführen.

Für einen oder beide Anschlüsse zum Hämodialysator wie auch für andere Anschlüsse, insbesondere zu verwendeter Auswertungs-Hardware, können mechanische- und/oder elektrische bzw. elektronische Anschlußkodierungen vorgesehen sein, welche sicherstellen, daß kompatible Geräte verwendet sowie Anschlüsse der arteriellen und der venösen Seite nicht vertauscht werden. Ferner können geeignete Anschlußkodierungen vom Hämodialysator sowie von angeschlossener Auswertungs-Hardware abgefragt werden, um Betriebs-, Bedienungs-, Korrektur- oder Berechnungsparameter an den verwendeten Typ der erfindungsgemäßen Vorrichtung anzupassen. Vorteilhaft lassen sich entsprechende Kodierungen beispielsweise als Widerstände, Impedanzbrücken, elektrisch angeschlossene oder transpondergekoppelte Chips, bestimmte Pin-Anordnungen oder dergleichen mehr realisieren.

Der im arteriellen Anschlußstück vorgesehene Sensor kann vorteilhafterweise wie bekannte in Dilutionsmeßverfahren eingesetzte Sensoren ausgeführt sein. Für die Temperaturmessung ist insbesondere ein Platinwiderstandssensor geeignet, daneben sind jedoch auch andere Thermowiderstände oder Thermoelemente zweckgemäß.

Der Injektionskanal des venösen Anschlußstücks der erfindungsgemäßen Vorrichtung kann vorteilhafterweise Ausstattungsmerkmale des aus der Druckschrift EP 1 034 737 A1 bekannten Injektionskanals aufweisen, alternativ aber auch einfacher gestaltet sein. Während dieser vor allem für die Verwendung eines Injektats mit Raumtemperatur optimiert ist, können erfindungsgemäß alternativ auch gekühlte oder erwärmte Injektate verwendet werden.

Vorzugsweise ist dabei die programmtechnische Einrichtung der Auswerteeinheit zur Durchführung von Auswerteschritten eines Thermodilutionsverfahrens gemäß einer der oben angeführten Druckschriften ausgestaltet.

Die Bestimmung des Injektionszeitpunkts und der Injektionsdauer kann vorteilhafterweise vorgesehen sein, wie in der deutschen Offenlegungsschrift DE 197 38 942 A1 beschrieben. Dies ist jedoch nicht unbedingt erforderlich.

Gemäß einer besonders vorteilhaften Weiterbildung der vorliegenden Erfindung ist ein Regelkreis vorgesehen, welcher die Einwirkung auf Flüssigkeitsentzug bzw. Flüssigkeitsanreicherung des Bluts im Hämodialysator in Abhängigkeit ermittelter Änderungen des Kreislauffüllzustands bewirkt.

Grundsätzlich kann jede im Rahmen der vorliegenden Anmeldung beschriebene bzw. angedeutete Variante der Erfindung besonders vorteilhaft sein, je nach wirtschaftlichen und technischen Bedingungen im Einzelfall. Soweit nichts gegenteiliges dargelegt ist, bzw. soweit grundsätzlich technisch realisierbar, sind einzelne Merkmale der beschriebenen Ausführungsformen austauschbar oder miteinander kombinierbar.

Nachfolgend werden anhand der zugehörigen Zeichnungen Beispiele bevorzugter Ausführungsformen der vorliegenden Erfindung näher erläutert.

Die Zeichnungen sind dabei rein schematische und, aus Gründen der Anschaulichkeit, nicht maßstäbliche Darstellungen. Insbesondere können Verhältnisse zwischen den Abmessungen, vor allem Kanaldurchmesser, Schlauchlängen und Außenabmessungen von tatsächlichen Ausführungen abweichen. In der Praxis können die Abmessungen anhand der Anforderungen im Einzelfall sowie anhand gängiger Standardteile, wie Gefäßzugängen herkömmlicher Hämodialysatoren und im Markt befindlicher Injektionskanäle dimensioniert werden.

Einander entsprechende Elemente sind in den einzelnen Figuren jeweils, soweit sinnvoll, mit denselben Bezugzeichen versehen.

Fig.1    zeigt die Schnittdarstellung einer erfindungsgemäßen Vorrichtung, bei welcher arterielles und venöses Anschlußstück separat ausgeführt und jeweils bereits mit einer Fistula-Nadel als Gefäßzugang verbunden sind.

Fig.2    zeigt die Schnittdarstellung einer erfindungsgemäßen Vorrichtung, bei welcher arterielles und venöses Anschlußstück in eine gemeinsame Funktionseinheit integriert sind.

Fig.3    zeigt ein erfindungsgemäßes System mit der, nur stilisiert dargestellten, Vorrichtung aus Fig. 2, einem Hämodialysator und einer Auswerteeinheit, wobei die Vorrichtung über zwei Fistula-Nadein am Kreislauf eines Dialysepatienten angeschlossen ist.

Fig.4a    zeigt eine erfindungsgemäße Vorrichtung ähnlich Fig. 2, wobei jedoch für den arteriellen Gefäßanschluß ein Katheter vorgesehen ist und der Temperatursensor in den Katheter verlegt ist. Der Katheter ist unterbrochen und ungeschnitten dargestellt.

Fig.4b-c    illustrieren anhand einer gegenüber Fig. 4a vergrößerten Schnittdarstellung des distalen Katheterendes zwei verschiedene Varianten der Temperatursensoranordnung.

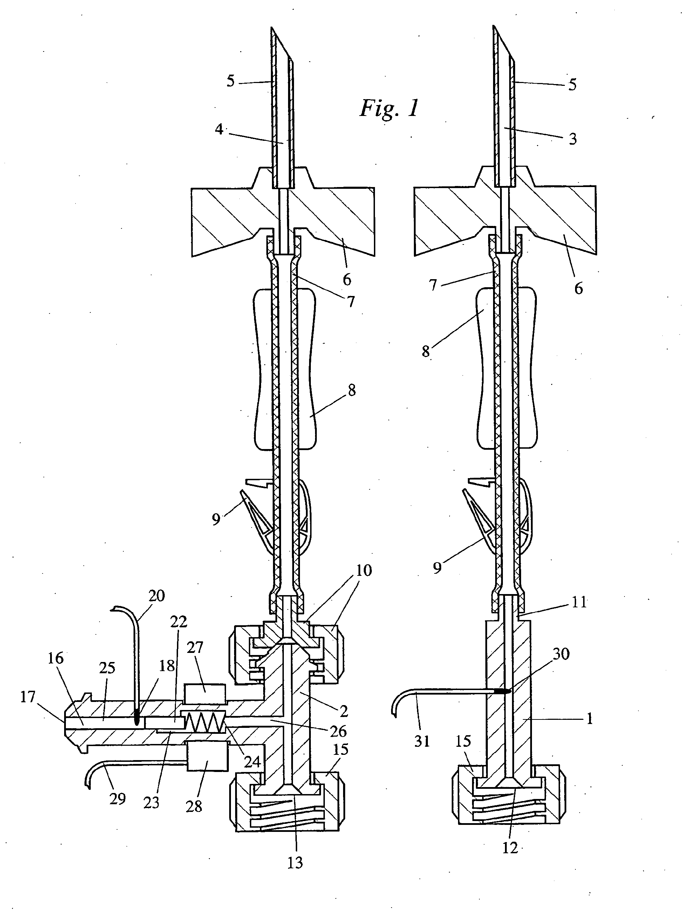

In Fig. 1 ist eine erfindungsgemäße Vorrichtung dargestellt, deren arterielles Anschlußstück 1 und venöses Anschlußstück 2 als separate Bauteile ausgeführt sind. Das arterielle Anschlußstück 1 ist mit einer arteriellen Fistula-Nadel 3 und das venöse Anschlußstück 2 mit einer venösen Fistula-Nadel 4 als Gefäßzugang verbunden. Die Fistula-Nadeln 3, 4 weisen jeweils eine angeschliffene Hohlnadel 5, ein Kunststoffgriffstück 6, sowie ein Schlauchstück 7 mit einer Schlauchhülse 8 und einer Quetschklemme 9 auf. Das Kunststoffgriffstück 6 ist vorzugsweise farblich gekennzeichnet, z.B. rot für den arteriellen und blau für die venösen Gefäßzugang.

Die Verbindung zwischen Anschlußstücken 1, 2 und Fistula-Nadeln 3, 4 kann beispielsweise über eine einfache Schlauchstutzenverbindung 11, wie für das arterielle Anschlußstück 1 dargestellt, oder auch auf andere Weise, etwa über eine Luer-Lock-Verbindung 10 oder andere Schnell-Verbindung, wie für das venöse Anschlußstück 2 dargestellt, ausgeführt sein. Der Hämodialysatoreingangsanschluß 12 am arteriellen Anschlußstück 1 und der Hämodialysatorausgangsanschluß 13 am venösen Anschlußstück 2 sind vorzugsweise ebenfalls so gestaltet, daß sich eine Schnell-Verbindung mit den Anschlüssen eines Hämodialysators 14, beispielsweise über Luer-Lock Muttern 15 herstellen läßt.

Das venöse Anschlußstück 2 weist einen Injektionskanal 16 auf, durch welchen ein zur Vornahme einer Dilutionsmessung benötigter Bolus in das Blut injiziert werden kann, welches das venöse Anschlußstück 2 vom Hämodialysatorausgangsanschluß 13 her kommend in Richtung des venösen Gefäßzugangs durchströmt. Nicht dargestellt ist ein Ventil, welches optional im Bereich des Hämodialysatorausgangsanschlusses 13 vorgesehen sein kann, um zu vermeiden, daß bei ausgeschaltetem Hämodialysator (bzw. bei Erliegen des Blutstroms 39 durch den Hämodialysator 14) Injektat durch den Hämodialysatorausgangsanschluß 13 in Richtung Hämodialysator gelangt. Hierbei kann es sich um ein einfaches Rückschlagventil, alternativ aber auch um ein (halb-)automatisch oder manuell betätigtes Ventil handen, beispielsweise ein von einem angeschlossenen Hämodialysator 14 elektronisch angesteuertes Magnetventil, welches bei Abschaltung des Hämodialysators 14 schließt.

Der dargestellte Injektionskanal 16 weist einen Anschluß 17 auf, an welchen eine Spritze, Injektionspumpe oder sonstige Vorrichtung zur Injektatzuführung angeschlossen werden kann. Eine Injektionspumpe kann vorteilhafterweise über eine Vorrichtung angesteuert werden, welche auch als Auswerteeinheit 21 für die Injektionsmessungen dient.

Ferner ist der Injektionskanal 16 mit einem Temperatursensor 18 zur Bestimmung der Injektat-Temperatur ausgestattet. Dargestellt ist lediglich rein schematisch die in den Injektionskanal 16 hineinragende Spitze eines Platin-Thermowiderstandsensors mit einer (nur angedeuteten) Signalleitung 20, welche in einem (eventuell geschirmten) Kabel verläuft und über einen (eventuell mechanisch und/oder elektrisch bzw. elektronisch kodierten) Stecker (nicht dargestellt) an eine Auswerteeinheit 21 angeschlossen werden kann. Ferner weist der Injektionskanal 16 ein Ventil auf, welches bei Überschreiten eines Mindestinjektionsdrucks öffnet und zugleich eine Schaltfunktion ausübt, über welche der Injektionszeitpunkt und/oder die Injektionsdauer bestimmt wird.

Das Ventil besteht im wesentlichen aus einem im Inneren des Injektionskanals 16 geführten Zylinder 22, einer oder mehrerer Längsnut(en) 23 im Inneren des Injektionskanals 16, welche sich nur über einen Teil der Zylinderlänge erstrecken, und einer Druckfeder 24, welche den Zylinder 22 gegen die Injektionsrichtung drückt. Vorzugsweise ist ein (nicht dargestellter) Anschlag vorgesehen, an welchem der Zylinder 22 anliegt, wenn kein oder nur ein geringer Injektionsdruck im Injektionskanal 16 herrscht. Die Druckfeder 24 ist dann vorzugsweise leicht vorgespannt. Steigt der Injektionsdruck, wird die Feder 24 komprimiert. Bei Überschreitung eines Schwellenwerts ist der Zylinderhub groß genug, daß die Längsnut 23 eine für das Injektat durchgängige Verbindung zwischen dem proximalen 25 und dem distalen 26 Teil des Injektionskanals 16 herstellt, und das Injektat so in das vom Hämodialysatorausgangsanschluß 13 her in Richtung des venösen Gefäßzugangs strömende Blut eintreten kann. Am Ende der Injektion drückt die Rückstellkraft der Feder 24 den Kolben 22 zurück in seine Ausgangsposition.

Um die Schaltfunktion zur Bestimmung des Injektionszeitpunkts und/oder der Injektionsdauer (vorzugsweise beider) auszuüben, besteht der Zylinder 22 aus ferromagnetischem Metall und wirkt mit einem Magneten 27 und einem Reed-Schalter 28 zusammen. Befindet sich der Zylinder 22 (während der Injektion) zwischen Magnet 27 und Reed-Schalter 28, so wird das magnetische Feld abgelenkt. Befindet sich der Zylinder 22 dagegen nicht zwischen Magnet 27 und Reed-Schalter 28 (vor und nach der Injektion), wird der Reed-Schalter 28 durch Wirkung des magnetischen Feldes betätigt. Die Abfrage des Reed-Schalters 28 über eine (in der Zeichnung nur angedeutete) Signalleitung 29 ermöglicht die Ermittlung von Injektionszeitpunkt und/oder Injektionsdauer (vorzugsweise von beidem). Die Signalleitung 29 kann (eventuell gemeinsam mit der Signalleitung 20 des Temperatursensors 18) in einem (eventuell geschirmten) Kabel verlaufen und über einen (eventuell mechanisch und/oder elektrisch bzw. elektronisch kodierten) Stecker (nicht dargestellt) an die Auswerteeienheit 21 angeschlossen werden.

Das Ventil bzw. der Schalter kann selbstverständlich auch anders ausgeführt sein, als vorliegend dargestellt. Beispielsweise können Zeitpunkt und Dauer der Injektion auch über eine kapazitive Messung oder über die Temperaturmessung bestimmt werden. Ferner kann der Injektionszeitpunkt manuell festgehalten werden, z.B. über einen Taster, welcher von die Injektion vornehmendem Personal bedient wird. Je nach Anspruch an die Güte der Meßtechnik ist auch eine einfachere Ausführung des Injektionskanals 16 ohne Ventil bzw. Schalter und/oder ohne Sensor möglich. Auch ein beheizbarer Injektionskanal 16 ist realisierbar.

Das arterielle Anschlußstück 1 weist einen Temperatursensor 30 auf, durch welchen die Temperatur des Bluts gemessen werden kann, welches das venöse Anschlußstück 2 vom arteriellen Gefäßzugang her kommend in Richtung des Hämodialysatoreingarigsanschlusses 12 durchströmt. Über diese Temperaturmessung läßt sich die Systemantwort auf die mittels der Bolusinjektion dem Kreislaufsystem des Dialysepatienten 32 zugeführte Störung bestimmen.

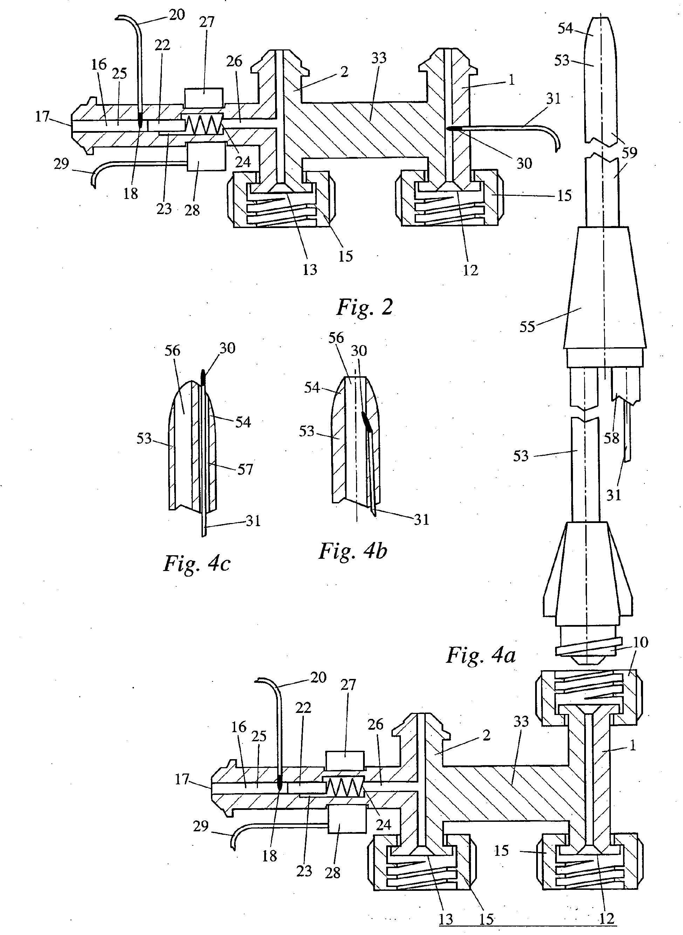

Dargestellt ist lediglich rein schematisch die in das Innere des arteriellen Anschlußstücks 1 hineinragende Spitze eines Platin-Thermowiderstandsensors mit einer (nur angedeuteten) Signalleitung 31, welche in einem (eventuell geschirmten) Kabel verläuft und über einen (eventuell mechanisch und/oder elektrisch bzw. elektronisch kodierten) Stecker (nicht dargestellt) an die Auswerteeienheit 21 angeschlossen werden kann. Bei Integration von arteriellem und venösem Änschlußstück (1, 2) in eine gemeinsame Funktionseinheit, wie in Fig. 2 dargestellt, können auch alle Signalleitungen 20, 29, 31 in einem gemeinsamen Kabel verlaufen.

Vorzugsweise sind die Anschlußstücke 1, 2 aus hygienischen Gründen als Einweg-Produkte ausgeführt und vor der Anwendung einzeln oder zusammen, mit oder ohne Fistula-Nadeln 3, 4 steril verpackt.

Aufbau und Funktionsweise der in Fig. 2 dargestellten erfindungsgemäßen Vorrichtung ist im wesentlichen gleich wie anhand Fig. 1 erläutert. Arterielles und venöses Anschlußstück (1, 2) sind jedoch als gemeinsame Funktionseinheit 33 miteinander verbunden. Für beide Gefäßzugänge sind Schnellverschlüsse zum Anschluß von Fistula-Nadeln (3, 4) vorgesehen.

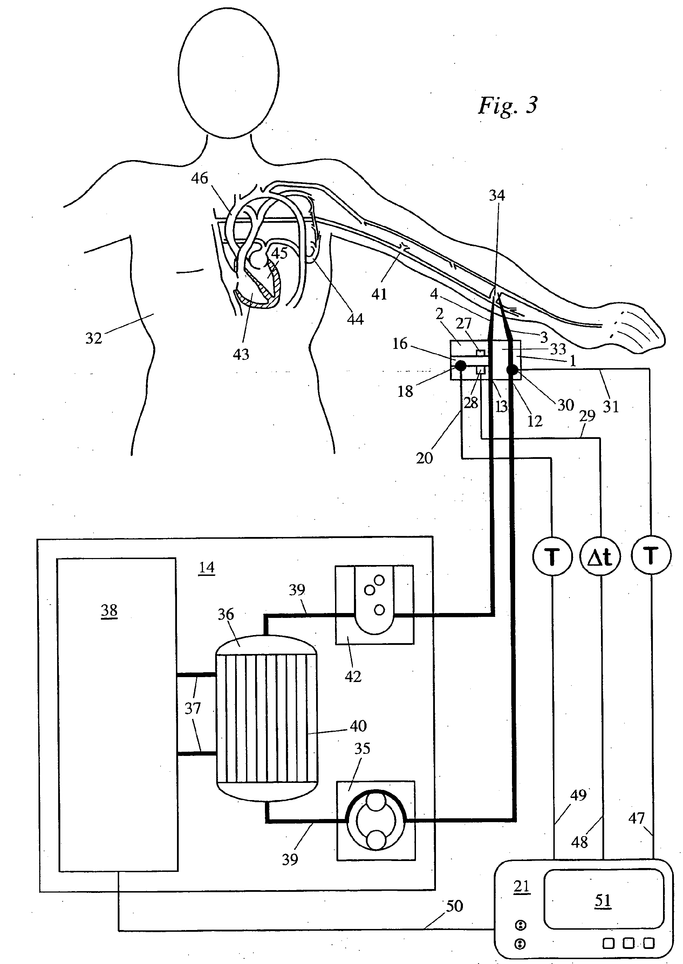

Fig. 3 zeigt ein erfindungsgemäßes System, bei welchem eine gemäß Fig. 2 oder ähnlich gestaltete Vorrichtung 33 an einen Hämodialysator 14 angeschlossen ist. Die Signalleitungen 20, 29 und 31 sind mit der Auswerteeienheit 21 verbunden, welche programmtechnisch zur Auswertung der Thermodilutionsmessungen ausgestattet ist, welche mit Hilfe der erfindungsgemäßen Vorrichtung 33 durchgeführt werden können.

Mittels Fistula-Nadein (3, 4) sind ein arterieller und ein venöser Gefäßzugang an einer arteriovenösen Fistel 34 eines Dialysepatienten 32 hergestellt, welche jeweils mit dem arteriellen bzw. venösen Anschlußstück (1, 2) verbunden sind.

Vom arteriellen Gefäßzugang fließt zu dialysierendes Blut, gefördert von einer Blutpumpe 35, durch das arterielle Anschlußstück 1 dem Hämodialysator 14 zu. In aller Regel wird das Blut heparinisiert. Im eigentlichen Dialysator 36, welcher mit einer großen Membranfläche 40 ausgestattet ist, erfolgt der transmembrane Stoffaustausch mit dem Permeat, und somit die "Blutwäsche". Der Permeatstrom 37 durchläuft ein aufwendiges, hier nicht näher dargestelltes Bilanzierungssystem 38, welches grundsätzlich wie in herkömmlichen Hämodialysatoren ausgeführt sein kann. In dem Bilanzierungssystem 38 werden Elektrolyt- und Flüssigkeitsgehalt des Permeats genau eingestellt, ferner wird das Permeat ultrafiltriert, mittels Wärmetauschern temperiert, entgast und auf Leckage-Blut untersucht.

Nach Überströmen der Membranfläche 40 im eigentlichen Dialysator 36 wird der Blutstrom 39 durch eine Luftfalle 42 geführt und tritt dann durch den Hämodialysatorausgangsanschluß 13 in das venöse Anschlußstück 2 ein. Von diesem gelangt das Blut durch den venösen Gefäßzugang zurück in den Körperkreislauf 41 des Patienten 32.

Im venösen Anschlußstück 2 kann über den Injektionskanal 16 ein Bolus injiziert werden, dessen Temperatur sich von der Bluttemperatur unterscheidet. Vorzugsweise werden Temperatur, Zeitpunkt und gegebenenfalls auch Dauer der Bolusinjektion auf oben beschriebene Weise mit Hilfe des Temperatursensors 18 und des Reed-Schalters 28 gemessen und die Meßwerte der Auswerteeienheit 21 zugeführt.

Die dem Patientenblut solcherart aufgeprägte lokale Temperaturänderung setzt sich mit Fließrichtung des Blutes fort und erreicht nacheinander rechtes Atrium, rechten Ventrikel 43, Lungenkreislauf 44, linkes Atrium, linken Ventrikel 45 und über die Aorta 46 erneut den Körperkreislauf 41 des Patienten 32. Mittels des Temperatursensors 30 im arteriellen Anschlußstück 1 läßt sich so die Sytemantwort auf die durch die Bolusinjektion hervorgerufene Störung erfassen. Aus dem Temperaturverlauf (bzw. dem Verlauf der Temperaturabweichung von der normalen Bluttemperatur) über der Zeit ergibt sich die Thermodilutionskurve.

Aufbauend auf an sich bekannten Ansätzen der transpulmonalen Thermodilutionstechnik kann die Auswerteeienheit 21 somit verschiedene hämodynamische Parameter, insbesondere jedoch das globale enddiastolische Volumen GEDV und somit den Füllzustand des Patienten 32 berechnen. Über die Eingangskanäle 47, 48, 49 erhält die Auswerteeienheit 21 hierzu die arteriellen Temperaturmeßdaten von dem Temperatursensor 30 sowie die die Bolusinjektion charakterisierenden Meßgrößen Injektat-Temperatur, Injektionsdauer und Injektionszeitpunkt.

Das globale enddiastolische Volumen GEDV kann nach der Gleichung

$$GEDV = CO \cdot (MTT - DST)$$

bestimmt werden. Darin ist CO das Herzzeitvolumen (*Cardiac Output*), MTT die mittlere Durchgangszeit (*Mean Transit Time*) und DST die exponentielle Abkling- oder Abfallzeit (*Dowslope Time*), d.h. die Zeit, welche die auf die Bolusinjektion zurückzuführende Temperaturabweichung benötigt, um um den Faktor 1/e abzusinken.

Das Herzzeitvolumen CO kann durch Algorithmen bestimmt werden, denen die Stewart-Hamilton-Gleichung zugrundeliegt:

$$CO = \frac{V_L(T_B - T_L)K_1 K_2}{\int \Delta T_B(t)dt}$$

Darin ist $T_B$ die anfängliche Bluttemperatur, $T_L$ die Temperatur des als Thermoindikator verwendeten Bolus, also die gemessene Injektättemperatur, $V_L$ das Volumen des injizierten Bolus und $\Delta T_B(t)$ die Abweichung der Bluttemperatur von der Basislinientemperatur $T_B$ als Funktion der Zeit t. $K_1$ und $K_2$ sind empirisch zu bestimmende oder abzuschätzende Konstanten zur Berücksichtigung der spezifischen Meßanordnung.

Über das auch der Bedienerführung dienende Display 52 können die ermittelten hämodynamische Parameter ausgegeben werden. Ferner kann die Auswerteeinheit 21 auch mit einem externen Speichermedium und oder einem Drucker ausgestattet sein.

Über den Ausgangskanal 51 kann ferner das Bilanzierungssystem 38 angesteuert werden. Bei übermäßigen Abweichungen des globalen enddiastolischen Volumens GEDV von einem patientenspezifischen Sollwert kann so über gezielte Anhebung oder Absenkung des Flüssigkeitsgehalts eine Korrektur des Füllzustands des Patienten 32 erreicht werden. Eine entsprechende Steuerung ist optional. Alternativ kann der behandelnde Arzt auf Grundlage des ausgegebenen berechneten GEDV-Werts entsprechende Gegenmaßnahmen treffen.

Eine alternative Ausgestaltung der arteriellen Seite ist in zwei verschiedenen Varianten anhand Fig. 4a-c illustriert. Für den arteriellen Gefäßzugang ist hier ein Katheter 53 vorgesehen, welcher in Fig. 4a ungeschnitten, vom arteriellen Anschlußstück 1 getrennt und aus Platzgründen unterbrochen dargestellt ist. In den Figuren 4b und 4c sind anhand jeweils einer gegenüber Fig. 4a vergrößerten Schnittdarstellung des distalen Katheterendes 54 zwei verschiedene Varianten der Anordnung des Temperatursensors 30 illustriert. Die venöse.

Seite ist wie in Fig. 2 gestaltet.

Der Katheter 53 ist an seinem proximalen Ende über einer Luer-Lock-Verbindung 10 oder ähnliche Schnellverbindung an dem arteriellen Anschlußstück 1 anschließbar. Daneben kann es auch vorteilhaft sein, Katheter 53 und arterielles Anschlußstück 1 als gemeinsames Teil dauerhaft miteinander zu verbinden, beispielsweise durch eine Verschweißung oder Verklebung.

Vom proximalen Ende des Katheters 53 verläuft ein Blutzuführungslumen 56 bis zum distalen Katheterende 54. Durch das Blutzuführungslumen 56 wird zu dialysierendes arterielles Blut dem arteriellen Anschlußstück 1 zugeführt.

Distal von der Kanaltrennung 55 verlaufen die Signalleitung 31 des Temperatursensors 30 und das Blutzuführungslumen 56 in einem gemeinsamen Katheterkörper 59. Der Temperatursensor 30 kann fest in den Katheter 53 integriert sein, wie in Fig. 4b dargestellt ist, oder aber als Teil einer separaten Sonde ausgeführt sein, welche durch ein Sondenlumen 57 eingeführt wird, wie in Fig. 4c dargestellt. Gemäß einer weiteren, nicht dargestellten, Variante kann der Temperatursensor 30 auch als separate Sonde ausgeführt sein, welche im Blutzuführungslumen 56 geführt wird.

Die Messung der Temperatur des zu dialysierenden Bluts kann entweder, wie in Fig. 4c dargestellt, im freien Blutfluß erfolgen, indem der Temperatursensor 30 über das distale Katheterende 54 hinausragt, oder aber im Inneren des Blutzuführungslumens 56, wie in Fig. 4b dargestellt.

Proximal von der Kanaltrennung 55 verläuft die Signalleitung 31 in einem eigenen Schlauchstück oder Kabel 58 und ist über einen (eventuell mechanisch und/ oder elektrisch bzw. elektronisch kodierten) Stecker (nicht dargestellt) an die Auswerteeinheit 21 anschließbar.

**Patentansprüche**

1. System zur Vornahme von Dilutionsmessungen am Herzkreislaufsystem eines Dialysepatienten (32), welches folgendes aufweist:

   - ein venöses Anschlußstück (2), aufweisend einen Hämodialysatorausgangsanschluß (13) und einen Injektionskanal (16) zum Injizieren eines Bolus in dialysiertes, das venöse Anschlußstück (2) durchfließendes Blut,
   - ein arterielles Anschlußstück (1), aufweisend einen Hämodialysatoreingangsanschluß (12),
   - einen Sensor (30) zur Messung der Temperatur zu dialysierenden Bluts, sowie
   - eine Auswerteeinheit (21), welche

   einen Eingangskanal (47) zum Einlesen eines Meßsignals des Sensors (30) zur Messung der Temperatur zu dialysierenden Bluts sowie

einen weiteren Eingangskanal (48, 49) zum Einlesen und/oder eine Eingabeeinheit zum Eingeben von mindestens einer eine Bolusinjektion charakterisierenden Größe aufweist,

**dadurch gekennzeichnet, dass** die Auswerteeinheit (21) programmtechnisch dazu ausgelegt ist, eine transpulmonale Thermodilutionsmessung auszuwerten, indem ausgehend von dem eingelesenen Meßsignal und der mindestens einen eine Bolusinjektion charakterisierenden Größe mindestens ein hämodynamischer Parameter bestimmt wird und der Sensor (30) im arteriellen Anschlußstück (1) vorgesehen ist.

2. System gemäß Anspruch 1 wobei das arterielle Anschlußstück (1) Verbindungsmittel zum Verbinden mit einem arteriellen Gefäßzugang und das venöse Anschlußstück Verbindungsmittel zum Verbinden mit einem venösen Gefäßzugang aufweist.

3. System gemäß Anspruch 2, wobei der arterielle Gefäßzugang über die Verbindungsmittel des arteriellen Anschlußstücks mit dem arteriellen Anschlußstück und der venöse Gefäßzugang über die Verbindungsmittel des venösen Anschlußstücks mit dem venösen Anschlußstück verbunden ist.

4. System gemäß einem der Ansprüche 2-3, wobei der venöse Gefäßzugang eine Fistula-Nadel (4) aufweist.

5. System gemäß einem der Ansprüche 2-4, wobei der arterielle Gefäßzugang eine Fistula-Nadel (3) aufweist.

6. System gemäß einem der Ansprüche 2-3, wobei der arterielle Gefäßzugang und der venöse Gefäßzugang in einen Shunt integriert sind.

7. System zur Vornahme von Dilutionsmessungen am Herzkreislaufsystem eines Dialysepatienten (32), welche folgendes aufweist:

   - ein venöses Anschlußstück (2), aufweisend einen Hämodialysatorausgangsanschluß (13) und einen Injektionskanal (16) zum Injizieren eines Bolus in dialysiertes, das venöse Anschlußstück (2) durchfließendes Blut,
   - ein arterielles Anschlußstück (1), aufweisend einen Hämodialysatoreingangsanschluß (12) und Verbindungsmittel, über welche das arterielle Anschlußstück (1) mit einem arteriellen Gefäßzugang verbunden ist,
   - einen Sensor (30) zur Messung der Temperatur zu dialysierenden Bluts, sowie
   - eine Auswerteeinheit (21), welche

   einen Eingangskanal (47) zum Einlesen eines Meßsignals des Sensors (30) zur Messung der Temperatur zu dialysierenden Bluts sowie einen weiteren Eingangskanal (48, 49) zum Einlesen und/oder eine Eingabeeinheit zum Eingeben von mindestens einer eine Bolusinjektion charakterisierenden Größe aufweist,

**dadurch gekennzeichnet, dass** die Auswerteeinheit (21) programmtechnisch dazu ausgelegt ist, eine transpulmonale Thermodilutionsmessung auszuwerten, indem ausgehend von dem eingelesenen Meßsignal und der mindestens einen eine Bolusinjektion charakterisierenden Größe mindestens ein hämodynamischer Parameter bestimmt wird, und dass der arterielle Gefäßzugang einen Katheter (53) aufweist, und der Sensor (30) fest in den Katheter (53) integriert oder Teil einer seperaten Sonde ist, die in ein Lumen (57) des Katheters (53) eingeschoben ist.

8. System gemäß einem der Ansprüche 7, wobei der Sensor (30) zur Messung der Temperatur den Katheter durchfließenden Bluts ausgelegt ist.

9. System gemäß einem der Ansprüche 7, wobei der Sensor (30) zur Messung der Temperatur des freien Blutflusses in der Nähe des distalen Katheterendes (54) ausgelegt ist.

10. System gemäß einem der vorangehenden Ansprüche, wobei das arterielle Anschlußstück (1) und das venöse Anschlußstück (2) in eine gemeinsame Funktionseinheit (33) integriert sind.

11. System gemäß einem der vorangehenden Ansprüche, wobei der Injektionskanal (16) einen Temperatursensor (18) zur Messung der Temperatur im Inneren des Injektionskanals (16) aufweist.

12. System gemäß einem der vorangehenden Ansprüche, wobei der Injektionskanal (16) einen Druckschalter mit einer Schaltcharakteristik aufweist, welche eine Schalteröffnung oder einen Schalterschluß ab Erreichen eines Druckschwellenwerts im Injektionskanal (16) bewirkt.

13. System gemäß einem der vorangehenden Ansprüche, wobei der Injektionskanal (16) einen Strömungsschalter mit einer Schaltcharakteristik aufweist, welche eine Schalteröffnung oder einen Schalterschluß ab Erreichen eines Flüssigkeitsstromschwellenwerts im Injektionskanal (16) bewirkt.

14. System gemäß einem der vorangehenden Ansprüche, wobei der Injektionskanal (16) ein Ventil aufweist.

15. System gemäß einem der vorangehenden Ansprüche, wobei der Hämodialysatorausgangsanschluß (13) des venösen Anschlußstücks mit einem Ventil zur Vermeidung eines Bolusflusses in Richtung Hämodialysator (14) ausgestattet ist.

16. System gemäß einem der vorangehenden Ansprüche, wobei die programmtechnische Auslegung der Auswerteeinheit (21) eine Funktion zur Bestimmung des Füllzustands des Dialysepatienten (32) umfaßt.

17. System gemäß einem der vorangehenden Ansprüche, wobei die Auswerteeinheit (21) ferner einen Steuerkanal (50) zur Ansteuerung eines Hämodialysators (14) aufweist, sowie die programmtechnische Auslegung der Auswerteeinheit (21) eine Steuerfunktion zur Beeinflussung der Flüssigkeitsbilanz den Hämodialysator (14) durchströmenden Bluts in Abhängigkeit des Füllzustands aufweist.

18. System gemäß einem der vorangehenden Ansprüche, welches einen Hämodialysator (14) aufweist.

**Claims**

1. System for carrying out dilution measurements on the cardiovascular system of a dialysis patient (32), which comprises the following:

    - a venous connection piece (2), comprising a hemodialyzer output connector (13) and an injection channel (16) for injecting a bolus into dialyzed blood that flows through the venous connection piece (2),
    - an arterial connection piece (1), comprising a hemodialyzer input connector (12),
    - a sensor (30) for measuring the temperature of blood to be dialyzed, as well as
    - an evaluation unit (21), which comprises an input channel (47) for reading in a measurement signal of the sensor (30) for measuring the temperature of blood to be dialyzed, as well as

    another input channel (48, 49) for reading in and/or an input unit for inputting at least one characteristic variable characterizing a bolus injection, **characterized in that** the evaluation unit (21) is adapted, in terms of program technology, for evaluating a transpulmonary thermodilution measurement, wherein at least one hemodynamic parameter is determined proceeding from the read-in measurement signal and the at least one variable characterizing a bolus injection, and **in that** the sensor (30) is provided in the arterial connection piece (1).

2. System according to claim 1, wherein the arterial connection piece (1) has connection means for connecting to an arterial blood vessel access and the venous connection piece has connection means for connecting to a venous blood vessel access.

3. System according to claim 2, wherein the arterial blood vessel access is connected with the arterial connection piece by way of the connection means of the arterial connection piece, and the venous blood vessel access is connected with the venous connection piece by way of the connection means of the venous connection piece.

4. System according to one of claims 2-3, wherein the venous blood vessel access comprises a fistula needle (4).

5. System according to one of claims 2-4, wherein the arterial blood vessel comprises has a fistula needle (3).

6. System according to one of claims 2-3, wherein the arterial blood vessel access and the venous blood vessel access are integrated into a shunt.

7. System for carrying out dilution measurements on the cardiovascular system of a dialysis patient (32), which comprises the following:

    - a venous connection piece (2), comprising a hemodialyzer output connector (13) and an injection channel (16) for injecting a bolus into dialyzed blood that flows through the venous connection piece (2),
    - an arterial connection piece (1), comprising a hemodialyzer input connector (12) and connection means by which the arterial connection piece is connected with an arterial blood vessel access,
    - a sensor (30) for measuring the temperature of blood to be dialyzed, as well as
    - an evaluation unit (21), which comprises an input channel (47) for reading in a measurement signal of the sensor (30) for measuring the temperature of blood to be dialyzed, as well as

    another input channel (48, 49) for reading in and/or an input unit for inputting at least one characteristic variable characterizing a bolus injection, **characterized in that** the evaluation unit is adapted, in terms of program technology, for evaluating a transpulmonary thermodilution measurement, wherein at least one hemodynamic parameter is determined proceeding from the read-in measurement signal and the at least one variable characterizing a bolus injection, and **in that** the arterial blood vessel access comprises a catheter (53), and the sensor (30) is either integrated into the catheter (53) in a fixed manner or is part of a probe which is inserted into a lumen (57) of the catheter (53).

8. System according to claim 7, wherein the sensor (30) is adapted for measuring the temperature of blood flowing through the catheter.

9. System according to claim 7, wherein the sensor (30) is adapted for measuring the temperature of the free blood flow in the vicinity of the distal catheter end (54).

10. System according to one of the preceding claims, wherein the arterial connection piece (1) and the venous connection piece (2) are integrated into a joint functional unit (33).

11. System according to one of the preceding claims, wherein the injection channel (16) comprises a temperature sensor (18) for measuring the temperature in the interior of the injection channel (16).

12. System according to one of the preceding claims, wherein the injection channel (16) comprises a pressure switch having switching characteristics that bring about switch opening or switch closing once a pressure threshold value has been reached in the injection channel (16).

13. System according to one of the preceding claims, wherein the injection channel (16) comprises a flow switch having switching characteristics that bring about switch opening or switch closing once a fluid flow threshold value has been reached in the injection channel (16).

14. System according to one of the preceding claims, wherein the injection channel (16) comprises a valve.

15. System according to one of the preceding claims, wherein the hemodialyzer output connector (13) of the venous connection piece is equipped with a valve for avoiding a bolus flow in the direction of the hemodialyzer (14).

16. System according to one of the preceding claims, wherein the program technology design of the evaluation unit (21) includes a function for determining the filling status of the dialysis patient (32).

17. System according to one of the preceding claims, wherein the evaluation unit (21) furthermore comprises a control channel (50) for controlling a hemodialyzer (14), and the program technology design of the evaluation unit (21) comprises a control function for influencing the fluid balance of blood flowing through the hemodialyzer (14), depending on the filling status.

18. System according to one of the preceding claims, which comprises a hemodialyzer (14).

## Revendications

1. Système destiné à réaliser des mesures de dilution sur le système cardiovasculaire d'un patient dialysé (32), comprenant les éléments suivants :

   - une pièce de raccordement veineux (2), comprenant un raccordement de sortie (13) de l'hémodialyseur et un canal d'injection (16) destiné à injecter un bolus dans le sang dialysé traversant la pièce de raccordement veineux (2) ;
   - une pièce de raccordement artériel (1), comprenant un raccordement d'entrée (12) de l'hémodialyseur ;
   - un capteur (30) destiné à mesurer la température du sang à dialyser ; et
   - une unité d'analyse (21) qui comprend :

      - un canal d'entrée (47) destiné à lire un signal de mesure du capteur (30) afin de mesurer la température du sang à dialyser ; et
      - un autre canal d'entrée (48, 49) pour la lecture et/ou une unité d'entrée pour la saisie d'au moins une grandeur caractéristique d'une injection d'un bolus ;

   **caractérisé par le fait que** l'unité d'analyse (21) est programmable et conçue pour analyser une mesure de thermodilution transpulmonaire dans laquelle, à partir du signal de mesure lu et de ladite au moins une grandeur caractéristique de l'injection d'un bolus, au moins un paramètre hémodynamique est déterminé et **par le fait que** le capteur (30) est implanté dans la pièce de raccordement artériel (1).

2. Système selon la revendication 1, dans lequel la pièce de raccordement artériel (1) possède un moyen de liaison pour la relier à un point d'accès à un vaisseau artériel et la pièce de raccordement veineux (2) possède un moyen de liaison pour la relier à un point d'accès à un vaisseau veineux.

3. Système selon la revendication 2, dans lequel le point d'accès au vaisseau artériel est relié à la pièce de raccordement artériel via le moyen de liaison de la pièce de raccordement artériel et le point d'accès au vaisseau veineux est relié à la pièce de raccordement veineux via le moyen de liaison de la pièce de raccordement veineux.

4. Système selon l'une des revendications 2 et 3, dans lequel le point d'accès au vaisseau veineux possède une aiguille à fistule (4).

**5.** Système selon l'une des revendications 2 à 4, dans lequel le point d'accès au vaisseau artériel possède une aiguille à fistule (3).

**6.** Système selon l'une des revendications 2 et 3, dans lequel le point d'accès au vaisseau artériel et le point d'accès au vaisseau veineux sont intégrés dans un shunt.

**7.** Système destiné à réaliser des mesures de dilution sur le système cardiovasculaire d'un patient dialysé (32), comprenant les éléments suivantes :

- une pièce de raccordement veineux (2), comprenant un raccordement de sortie (13) de l'hémodialyseur et un canal d'injection (16) destiné à injecter un bolus dans le sang dialysé traversant la pièce de raccordement veineux (2) ;
- une pièce de raccordement artériel (1), comprenant un raccordement d'entrée (12) de l'hémodialyseur et un moyen de liaison grâce auquel la pièce de raccordement artériel (1) est reliée à un point d'accès au vaisseau artériel ;
- un capteur (30) destiné à mesurer la température du sang à dialyser ; et
- une unité d'analyse (21) qui comprend :

- un canal d'entrée (47) destiné à lire un signal de mesure du capteur (30) afin de mesurer la température du sang à dialyser ; et
- un autre canal d'entrée (48, 49) pour la lecture et/ou une unité d'entrée pour la saisie d'au moins une grandeur caractéristique d'une injection d'un bolus ;

**caractérisé par le fait que** l'unité d'analyse (21) est conçue pour être programmée afin d'analyser une mesure de thermodilution transpulmonaire dans laquelle, à partir du signal, de mesure lu et de ladite au moins une grandeur caractéristique de l'injection d'un bolus, au moins un paramètre hémodynamique est déterminé ;
et **par le fait que** le point d'accès au vaisseau artériel comprend un cathéter (53) et le capteur (30) est intégré à demeure dans le cathéter (53) ou est un élément d'une sonde séparée qui est insérée dans une lumière (57) du cathéter (53).

**8.** Système selon la revendication 7, dans lequel le capteur (30) est conçu pour mesurer la température du sang parcourant le cathéter.

**9.** Système selon la revendication 7, dans lequel le capteur (30) est conçu pour la mesure de la température du flux libre de sang à proximité de l'extrémité distale du cathéter (54).

**10.** Système selon l'une des revendications précédentes, dans lequel la pièce de raccordement artériel (1) et la pièce de raccordement veineux (2) sont intégrées dans une unité fonctionnelle commune (33).

**11.** Système selon l'une des revendications précédentes, dans lequel le canal d'injection (16) possède un capteur thermique (18) destiné à mesurer la température à l'intérieur du canal d'injection (16).

**12.** Système selon l'une des revendications précédentes, dans lequel le canal d'injection (16) possède un commutateur manométrique avec une caractéristique de commutation qui commande une ouverture du commutateur ou une fermeture du commutateur lorsqu'un seuil de pression est atteint dans le canal d'injection (16).

**13.** Système selon l'une des revendications précédentes, dans lequel le canal d'injection (16) possède un commutateur d'écoulement avec une caractéristique de commutation qui commande une ouverture du commutateur ou une fermeture du commutateur lorsqu'un seuil d'écoulement liquide est atteint dans le canal d'injection (16).

**14.** Système selon l'une des revendications précédentes, dans lequel le canal d'injection (16) possède une soupape.

**15.** Système selon l'une des revendications précédentes, dans lequel le raccordement de sortie (13) de l'hémodialyseur de la pièce de raccordement veineux est équipé d'une soupape afin d'éviter un écoulement du bolus dans la direction de l'hémodialyseur (14).

**16.** Système selon l'une des revendications précédentes, dans lequel la conception programmable de l'unité d'analyse (21) comprend une fonction de détermination de l'état de remplissage du patient dialysé (32).

**17.** Système selon l'une des revendications précédentes, dans lequel l'unité d'analyse (21) comprend en outre un canal de commande (50) pour piloter un hémodialyseur (14) et la conception programmable de l'unité d'analyse (21) comprend une fonction de commande afin d'affecter le bilan liquide du sang parcourant l'hémodialyseur (14) en fonction de l'état de remplissage.

**18.** Système selon l'une des revendications précédentes, comprenant un hémodialyseur (14).

*Fig. 1*

Fig. 2

Fig. 4c

Fig. 4b

Fig. 4a

*Fig. 3*

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4214402 A1
- DE 413093 A1
- US 6757554 B2
- DE 10143995 A1
- US 5595182 A
- EP 1034737 A1
- DE 19738942 A1